# EUROPEAN PATENT SPECIFICATION

(11) **EP 1 383 489 B1**
(45) Date of publication and mention of the grant of the patent: **09.05.2007**
(21) Application number: 02714977.2
(22) Date of filing: 21.02.2002
(51) Int. Cl.: A61K 31/27

(54) **CARBAMATE COMPOUNDS FOR USE IN PREVENTING OR TREATING NEURODEGENERATIVE DISORDERS**
CARBAMATVERBINDUNGEN ZUR VORBEUGUNG ODER BEHANDLUNG VON NEURODEGENERATIVEN STÖRUNGEN
COMPOSES CARBAMIQUES POUR LA PREVENTION OU LE TRAITEMENT DES MALADIES NEURODEGENERATIVES

(30) Priority: 27.02.2001 US 271682 P; 21.02.2002 US 81764
(43) Date of publication of application: 28.01.2004
(73) Proprietor: Ortho-McNeil Pharmaceutical, Inc., Raritan, NJ 08869-0602 (US)
(72) Inventor: PLATA-SALAMAN, Carlos R., Ambler, PA 19002 (US); ZHAO, Boyu, Landsdale, PA 19446 (US); TWYMAN, Roy E., Doylestown, PA 18901 (US)
(74) Representative: Fisher, Adrian John
(86) International application number: PCT/US2002/005541
(87) International publication number: WO 2002/067925

(56) References cited:
- US-A- 3 265 728
- US-A- 3 278 380
- US-A- 6 103 759

## Description

### Field of the Invention

This invention is directed to the use of a carbamate compound for the preparation of a medicament for preventing or treating neurodegenerative disorders. More particularly, this invention is directed to the use of halogenated 2-phenyl-1,2-ethanediol monocarbamate or dicarbamate compounds for the preparation of a medicament for preventing or treating neurodegenerative disorders.

### Background of the Invention

Acute and chronic neurodegenerative disorders are associated with neuronal cell death or compromise (McDonald ES, Windebank AJ, Mechanisms of neurotoxic injury and cell death, *Neurol. Clin.,* **2000**, Aug, 18(3), 525-40; Nagy Z, Mechanisms of neuronal death in Down's syndrome, J. *Neural. Transm. Suppl.,* **1999**, 57, 233-45; Kilpatrick TJ, Soilu-Hanninen M, Molecular mechanisms regulating motor neuron development and degeneration, *Mol. Neurobiol.,* **1999,** Jun, 19(3), 205-28; Rubin LL, Neuronal cell death: an updated view, *Prog. Brain. Res.,* **1998**, 117, 3-8; Saha AR, Ninkina NN, Hanger DP, Anderton BH, Davies AM, Buchman VL, Induction of neuronal death by alpha-synuclein, *Eur. J. Neurosci.,* **2000,** Aug, 12(8), 3073-3077; Varadarajan S, Yatin S, Aksenova M, Butterfield DA, Review: Alzheimer's amyloid beta-peptide-associated free radical oxidative stress and neurotoxicity, *J. Struct. Biol.,* **2000**, Jun, 130(2-3), 184-208; Clarke G, Collins RA, Leavitt BR, Andrews DF, Hayden MR, Lumsden CJ, Mcinnes RR, A one-hit model of cell death in inherited neuronal degenerations, *Nature,* **2000***,* Jul, 13, 406(6792), 195-9; Foley P, Riederer P, Influence of neurotoxins and oxidative stress on the onset and progression of Parkinson's disease, J. *Neurol.,* **2000,** Apr, 247 Suppl 2, 1182-94; Nicotera P, Caspase requirement for neuronal apoptosis and neurodegeneration, *IUBMB Life,* **2000**, May, 49(5), 421-5; Mattson MP, Pedersen WA, Duan W, Culmsee C, Camandola S, Cellular and molecular mechanisms underlying perturbed energy metabolism and neuronal degeneration in Alzheimer's and Parkinson's diseases, *Ann. N.Y. Acad. Sci*., **1999**, 893, 154-75; Martin LJ, Al-Abdulla NA, Brambrink AM, Kirsch JR, Sieber FE, Portera-Cailliau C, Neurodegeneration in excitotoxicity, global cerebral ischemia, and target deprivation: A perspective on the contributions of apoptosis and necrosis, *Brain Res. Bull.,* **1998**, Jul 1, 46(4), 281-309; McIntosh TK, Saatman KE, Raghupathi R, Graham Dl, Smith DH, Lee VM, Trojanowski JQ, The Dorothy Russell Memorial Lecture; The molecular and cellular sequelae of experimental traumatic brain injury: pathogenetic mechanisms, *Neuropathol. Appl. Neurobiol.,* **1998**, Aug, 24(4), 251-67). Prevention of neuronal cell death is required for the treatment of both acute and chronic neurodegenerative disorders.

Acute neurodegenerative disorders are those associated with an abrupt insult including, but not limited to, acute injury, hypoxia-ischemia or the combination thereof resulting in neuronal cell death or compromise. Acute injury includes, and is not limited to, brain trauma, focal brain trauma, diffuse brain damage, spinal cord injury, intracranial or intravertebral lesions (including, but not limited to, contusion, penetration, shear, compression or laceration lesions) or whiplash shaken infant syndrome. Hypoxia-ischemia includes, and is not limited to, cerebrovascular insufficiency, cerebral ischemia or cerebral infarction (including cerebral ischemias or infarctions originating from embolic occlusion and thrombotic occlusion, reperfusion following acute ischemia, perinatal hypoxic-ischemic injury, cardiac arrest or intracranial hemorrhage of any type (including, but not limited to, epidural, subdural, subarachnoid or intracerebral hemorrhage).

Chronic neurodegenerative disorders are those associated with progressive neuronal cell death or compromise over a period of time including, but not limited to, Alzheimer's disease, Pick's disease, diffuse Lewy body disease, progressive supranuclear palsy (Steel-Richardson syndrome), multisystem degeneration (Shy-Drager syndrome), chronic epileptic conditions associated with neurodegeneration, motor neuron diseases (amyotrophic lateral sclerosis), multiple sclerosis, degenerative ataxias, cortical basal degeneration, ALS-Parkinson's-Dementia complex of Guam, subacute sclerosing panencephalitis, Huntington's disease, Parkinson's disease, synucleinopathies (including multiple system atrophy), primary progressive aphasia, striatonigral degeneration, Machado-Joseph disease or spinocerebellar ataxia type 3 and olivopontocerebellar degenerations, bulbar and pseudobulbar palsy, spinal and spinobulbar muscular atrophy (Kennedy's disease), primary lateral sclerosis, familial spastic paraplegia, Werdnig-Hoffmann disease, Kugelberg-Welander disease, Tay-Sach's disease, Sandhoff disease, familial spastic disease, Wohlfart-Kugelberg-Welander disease, spastic paraparesis, progressive multifocal leukoencephalopathy, familial dysautonomia (Riley-Day syndrome) or prion diseases (including, but not limited to Creutzfeldt-Jakob disease, Gerstmann-Sträussler-Scheinker disease, Kuru disease or fatal familial insomnia).

Other acute or chronic neurodegenerative disorders associated with memory loss include, and are not limited to, neurodegenerative disorders associated with age-related dementia, vascular dementia, diffuse white matter disease (Binswanger's disease), dementia of endocrine or metabolic origin, dementia of head trauma and diffuse brain damage, dementia pugilistica or frontal lobe dementia.

Other acute or chronic neurodegenerative disorders associated with neuronal injury include, and are not limited to, neurodegenerative disorders associated with chemical, toxic, infectious and radiation injury of the nervous system, injury during fetal development, prematurity at time of birth, anoxic-ischemia, injury from hepatic, glycemic, uremic, electrolyte and endocrine origin, injury of psychiatric origin (including, but not limited to, psychopathology, depression or anxiety), injury from peripheral diseases and plexopathies (including plexus palsies) or injury from neuropathy (including neuropathy selected from multifocal, sensory, motor, sensory-motor, autonomic, sensory-autonomic or demyelinating neuropathies (including, but not limited to Guillain-Barre syndrome or chronic inflammatory demyelinating polyradiculoneuropathy) or those neuropathies originating from infections, inflammation, immune disorders, drug abuse, pharmacological treatments, toxins, trauma (including, but not limited to compression, crush, laceration or segmentation traumas), metabolic disorders (including, but not limited to, endocrine or paraneoplastic), Charcot-Marie-Tooth disease (including, but not limited to, type 1a, 1b, 2, 4a or 1-X linked), Friedreich's ataxia, metachromatic leukodystrophy, Refsum's disease, adrenomyeloneuropathy, Ataxia-telangiectasia, Déjerine-Sottas (including, but not limited to, types A or B), Lambert-Eaton syndrome or disorders of the cranial nerves).

Substituted phenyl alkyl carbamate compounds have been described in US Patent No. 3,265,728 to Bossinger, et al, as useful in treating the central nervous system, having tranquilization, sedation and muscle relaxation properties of the formula: 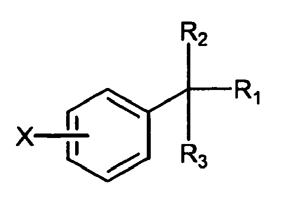 wherein R₁ is either carbamate or alkyl carbamate containing from 1 to 3 carbon atoms in the alkyl group; R₂ is either hydrogen, hydroxy, alkyl or hydroxy alkyl containing from 1 to 2 carbons; R₃ is either hydrogen or alkyl containing from 1 to 2 carbons; and X can be halogen, methyl, methoxy, phenyl, nitro or amino.

A method for inducing calming and muscle relaxation with carbamates has been described in US Patent No. 3,313,692 to Bossinger, et al by administering a compound of the formula: 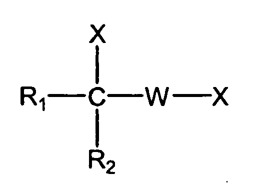 in which W represents an aliphatic radical containing less than 4 carbon atoms, wherein R₁ represents an aromatic radical, R₂ represents hydrogen or an alkyl radical containing less than 4 carbon atoms, and X represents hydrogen or hydroxy or alkoxy and alkyl radicals containing less than 4 carbon atoms or the radical: 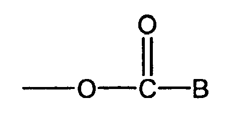 in which B represents an organic amine radical of the group consisting of heterocyclic, ureido and hydrazino radicals and the radical -N(R₃)₂ wherein R₃ represents hydrogen or an alkyl radical containing less than 4 carbon atoms.

Optically pure forms of halogen substituted 2-phenyl-1,2-ethanediol monocarbamates and dicarbamates have also been described in US Patent No. 6,103,759 to Choi, et al, as effective for treating and preventing central nervous system disorders including convulsions, epilepsy, stroke and muscle spasm; and as useful in the treatment of central nervous system diseases, particularly as anticonvulsants, antiepileptics, neuroprotective agents and centrally acting muscle relaxants, of the formulae: 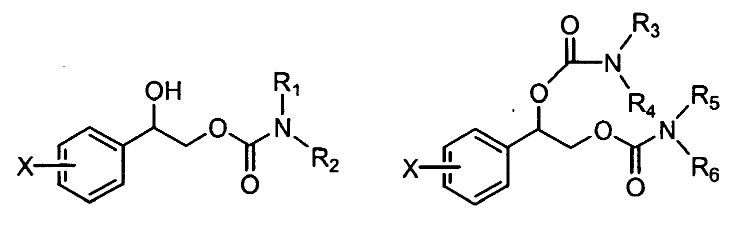 wherein one enantiomer predominates and wherein the phenyl ring is substituted at X with one to five halogen atoms selected from fluorine, chlorine, bromine or iodine atoms and R₁, R₂, R₃, R₄, R₅ and R₆ are each selected from hydrogen and straight or branched alkyl groups with one to four carbons optionally substituted with a phenyl group with substituents selected from the group consisting of hydrogen, halogen, alkyl, alkyloxy, amino, nitro and cyano. Pure enantiomeric forms and enantiomeric mixtures were described wherein one of the enantiomers predominates in the mixture for the compounds represented by the formulae above; preferably one of the enantiomers predominates to the extent of about 90% or greater; and, most preferably, about 98% or greater.

Halogen substituted 2-phenyl-1,2-ethanediol carbamate compounds of Formula (I) or Formula (II) have not been previously described as useful for preventing or treating neurodegenerative disorders. Recent preclinical studies have revealed previously unrecognized pharmacological properties which suggest that a compound of Formula (I) or Formula (II) is useful in preventing or treating neurodegenerative disorders. Therefore, it is an object of the present invention to teach a method for use of a compound of Formula (I) or Formula (II) in preventing or treating neurodegenerative disorders.

### Summary of the Invention

The present invention is directed to the use according to the claims of a therapeutically effective amount of a compound selected from the group consisting of Formula (I) and Formula (II): 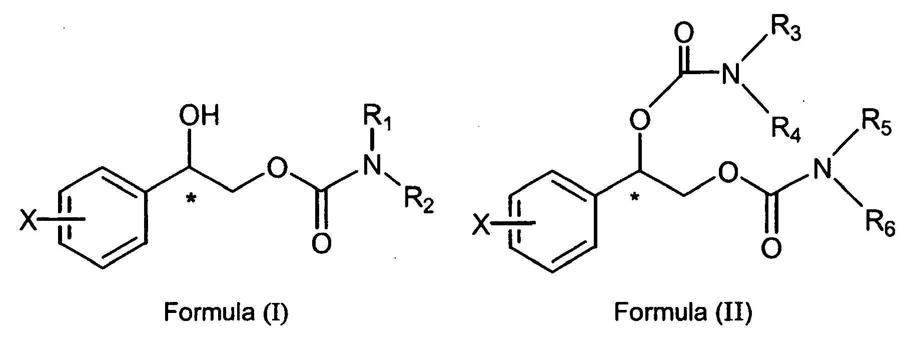 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano).

Embodiments of the invention include the use of a compound selected from the group consisting of Formula (I) and Formula (II) for the preparation of a medicament for preventing or treating neurodegenerative disorders in a subject in need thereof.

Embodiments of the method include the use of an enantiomer selected from the group consisting of Formula (I) and Formula (II) or enantiomeric mixture wherein one enantiomer selected from the group consisting of Formula (I) and Formula (II) predominates. For enantiomeric mixtures wherein one enantiomer selected from the group consisting of Formula (I) and Formula (II) predominates, preferably, one enantiomer selected from the group consisting of Formula (I) and Formula (II) predominates to the extent of about 90% or greater. More preferably, one enantiomer selected from the group consisting of Formula (I) and Formula (II) predominates to the extent of about 98% or greater.

### Detailed Description of the Invention

The present invention is directed to a use according to the claims of a therapeutically effective amount of a compound selected from the group consisting of Formula (I) and Formula (II): 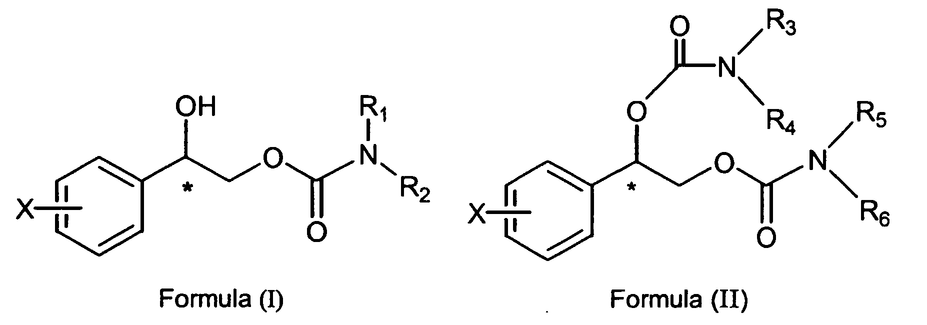 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano).

The present use includes the use of a compound selected from the group consisting of Formula (I) and Formula (II) wherein X is chlorine; preferably, X is substituted at the ortho position of the phenyl ring.

The present use also includes the use of a compound selected from the group consisting of Formula (I) and Formula (II) wherein R₁, R₂, R₃, R₄, R₅ and R₆ are preferably selected from hydrogen.

An embodiment of the present invention includes the use of an enantiomer selected from the group consisting of Formula (I) and Formula (II) or enantiomeric mixture wherein one enantiomer selected from the group consisting of Formula (I) and Formula (II) predominates wherein X is chlorine; preferably, X is substituted at the ortho position of the phenyl ring.

The present invention also includes the use of an enantiomer selected from the group consisting of Formula (I) and Formula (II) or enantiomeric mixture wherein one enantiomer selected from the group consisting of Formula (1) and Formula (II) predominates wherein R₁, R₂, R₃, R₄, R₅ and R₆ are preferably selected from hydrogen.

For enantiomeric mixtures wherein one enantiomer selected from the group consisting of Formula (I) and Formula (II) predominates, preferably, an enantiomer selected from the group consisting of Formula (I) and Formula (II) predominates to the extent of about 90% or greater. More preferably, an enantiomer selected from the group consisting of Formula (I) and Formula (II) predominates to the extent of about 98% or greater.

An embodiment of the present invention includes the use of an enantiomer selected from the group consisting of Formula (Ia) and Formula (IIa) or enantiomeric mixture wherein one enantiomer selected from the group consisting of Formula (Ia) and Formula (IIa) predominates: 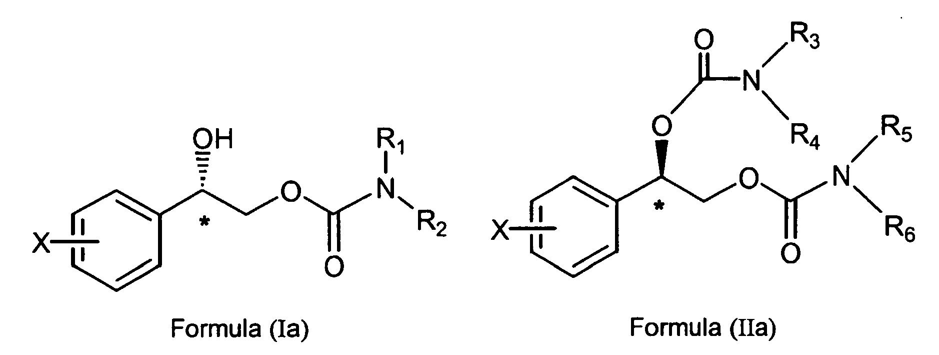 wherein
phenyl is substituted at X with one to five halogen atoms selected from the group consisting of fluorine, chlorine, bromine and iodine; and,
R_{1'} R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano).

The present invention includes the use of an enantiomer selected from the group consisting of Formula (Ia) and Formula (IIa) or enantiomeric mixture wherein one enantiomer selected from the group consisting of Formula (Ia) and Formula (IIa) predominates wherein X is chlorine; preferably, X is substituted at the ortho position of the phenyl ring.

The present invention also includes the use of an enantiomer selected from the group consisting of Formula (Ia) and Formula (IIa) or enantiomeric mixture wherein one enantiomer selected from the group consisting of Formula (Ia) and Formula (IIa) predominates wherein R₁, R₂, R₃, R₄, R₅, and R₆ are preferably selected from hydrogen.

For enantiomeric mixtures wherein one enantiomer selected from the group consisting of Formula (Ia) and Formula (IIa) predominates, preferably, an enantiomer selected from the group consisting of Formula (Ia) and Formula (IIa) predominates to the extent of about 90% or greater. More preferably, an enantiomer selected from the group consisting of Formula (Ia) and Formula (IIa) predominates to the extent of about 98% or greater.

An embodiment of the present invention includes a use according to the claims of therapeutically effective amount of an enantiomer selected from the group consisting of Formula (Ib) and Formula (IIb) or enantiomeric mixture wherein one enantiomer selected from the group consisting of Formula (Ib) and Formula (IIb) predominates:

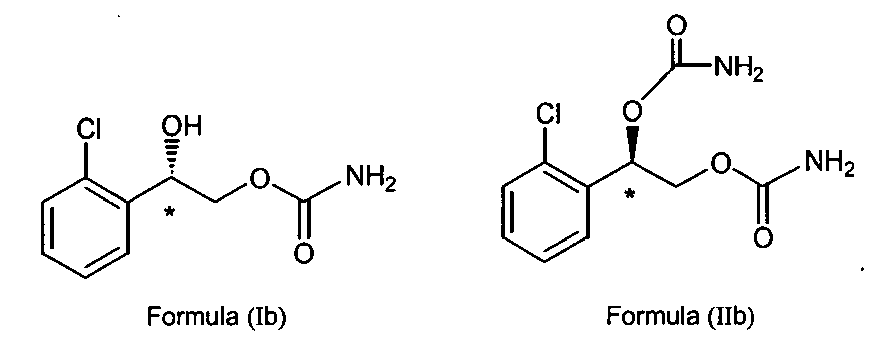

For enantiomeric mixtures wherein one enantiomer selected from the group consisting of Formula (Ib) and Formula (IIb) predominates, preferably, an enantiomer selected from the group consisting of Formula (Ib) and Formula (IIb) predominates to the extent of about 90% or greater. More preferably, an enantiomer selected from the group consisting of Formula (Ib) and Formula (IIb) predominates to the extent of about 98% or greater.

Other crystal forms of the present invention may exist and as such are intended to be included in the present invention.

It is apparent to those skilled in the art that the compounds of the invention are present as racemates, enantiomers and enantiomeric mixtures thereof. A carbamate enantiomer selected from the group consisting of Formula (I), Formula (II), Formula (Ia), Formula (IIa), Formula (Ib) and Formula (IIb) contains an asymmetric chiral carbon atom at the benzylic position, which is the aliphatic carbon adjacent to the phenyl ring (represented by the asterisk in the structural formulae).

Compounds of the present invention may be prepared as described in the previously referenced Bossinger '728 patent Bossinger'692 patent and Choi '759 patent).

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

The present invention contemplates a use according to the claims. Neurodegenerative disorders include, and are not limited to, acute neurodegenerative disorders, chronic neurodegenerative disorders, other acute or chronic neurodegenerative disorders associated with memory loss or other acute or chronic neurodegenerative disorders associated with neuronal injury.

Acute neurodegenerative disorders are those associated with an abrupt insult including, but not limited to, acute injury, hypoxia-ischemia or the combination thereof resulting in neuronal cell death or compromise. Acute injury includes, and is not limited to, brain trauma, focal brain trauma, diffuse brain damage, spinal cord injury, intracranial or intravertebral lesions (including, but not limited to, contusion, penetration, shear, compression or laceration lesions) or whiplash shaken infant syndrome. Hypoxia-ischemia includes, and is not limited to, cerebrovascular insufficiency, cerebral ischemia or cerebral infarction (including cerebral ischemias or infarctions originating from embolic occlusion or thrombotic occlusion, reperfusion following acute ischemia, perinatal hypoxic-ischemic injury, cardiac arrest or intracranial hemorrhage of any type (including, but not limited to, epidural, subdural, subarachnoid or intracerebral hemorrhage)).

Chronic neurodegenerative disorders are those associated with progressive neuronal cell death or compromise over a period of time including, but not limited to, Alzheimer's disease, Pick's disease, diffuse Lewy body disease, progressive supranuclear palsy (Steel-Richardson syndrome), multisystem degeneration (Shy-Drager syndrome), chronic epileptic conditions associated with neurodegeneration, motor neuron diseases (amyotrophic lateral sclerosis), multiple sclerosis, degenerative ataxias, cortical basal degeneration, ALS-Parkinson's-Dementia complex of Guam, subacute sclerosing panencephalitis, Huntington's disease, Parkinson's disease, synucleinopathies (including multiple system atrophy), primary progressive aphasia, striatonigral degeneration, Machado-Joseph disease / spinocerebellar ataxia type 3 and olivopontocerebellar degenerations, bulbar and pseudobulbar palsy, spinal and spinobulbar muscular atrophy (Kennedy's disease), primary lateral sclerosis, familial spastic paraplegia, Werdnig-Hoffmann disease, Kugelberg-Welander disease, Tay-Sach's disease, Sandhoff disease, familial spastic disease, Wohlfart-Kugetberg-Welander disease, spastic paraparesis, progressive multifocal leukoencephalopathy, familial dysautonomia (Riley-Day syndrome) or prion diseases (including, but not limited to Creutzfeldt-Jakob disease, Gerstmann-Straussler-Scheinker disease, Kuru disease or fatal familial insomnia).

Other acute or chronic neurodegenerative disorders associated with memory loss include, and are not limited to, neurodegenerative disorders associated with age-related dementia, vascular dementia, diffuse white matter disease (Binswanger's disease), dementia of endocrine or metabolic origin, dementia of head trauma and diffuse brain damage, dementia pugilistica or frontal lobe dementia.

Other acute or chronic neurodegenerative disorders associated with neuronal injury include, and are not limited to, neurodegenerative disorders associated with chemical, toxic, infectious and radiation injury of the nervous system, injury during fetal development, prematurity at time of birth, anoxic-ischemia, injury from hepatic, glycemic, uremic, electrolyte and endocrine origin, injury of psychiatric origin (including, but not limited to, psychopathology, depression or anxiety), injury from peripheral diseases and plexopathy (including plexus palsies) or injury from neuropathy (including neuropathy selected from multifocal, sensory, motor, sensory-motor, autonomic, sensory-autonomic or demyelinating neuropathies (including, but not limited to, Guillain-Barre syndrome or chronic inflammatory demyelinating polyradiculoneuropathy) or those neuropathies originating from infections, inflammation, immune disorders, drug abuse, pharmacological treatments, toxins, trauma (including, but not limited to, compression, crush, laceration or segmentation traumas), metabolic disorders (including, but not limited to, endocrine or paraneoplastic), Charcot-Marie-Tooth disease (including, but not limited to, type 1a, 1b, 2, 4a or 1-X linked), Friedreich's ataxia, metachromatic leukodystrophy, Refsum's disease, adrenomyeloneuropathy, Ataxia-telangiectasia, Déjerine-Sottas (including, but not limited to, types A or B), Lambert-Eaton syndrome or disorders of the cranial nerves).

An example of the method of the present invention comprises the use of a therapeutically effective amount of a compound selected from the group consisting of Formula (I) and Formula (II) in a pharmaceutical composition comprising a pharmaceutically acceptable carrier and a compound selected from the group consisting of Formula (I) and Formula (II). The method of the present invention also includes the use of a compound selected from the group consisting of Formula (I) and Formula (II) for the preparation of a medicament for preventing or treating neurodegenerative disorders.

Another example of the present invention comprises the use of a therapeutically effective amount of a compound selected from the group consisting of Formula (I) and Formula (II) or a pharmaceutical composition thereof in combination with one or more agents useful in preventing or treating neurodegenerative disorders.

A compound selected from the group consisting of Formula (I) and Formula (II) or pharmaceutical composition thereof may be administered by any conventional route of administration including, but not limited to oral, pulmonary, intraperitoneal (ip), intravenous (iv), intramuscular (im), subcutaneous (sc), transdermal, buccal, nasal, sublingual, ocular,, rectal and vaginal. In addition, administration directly to the nervous system may include, and are not limited to, intracerebral, intraventricular, intracerebroventricular, intrathecal, intracisternal, intraspinal or peri-spinal routes of administration by delivery via intracranial or intravertebral needles or catheters with or without pump devices. It will be readily apparent to those skilled in the art that any dose or frequency of administration that provides the therapeutic effect described herein is suitable for use in the present invention.

The therapeutically effective amount of a compound selected from the group consisting of Formula (I) and Formula (II) or pharmaceutical composition thereof may be from about 0.01 mg/Kg/dose to about 100 mg/Kg/dose. Preferably, the therapeutically effective amount may be from about 0.01 mg/Kg/dose to about 25 mg/Kg/dose. More preferably, the therapeutically effective amount may be from about 0.01 mg/Kg/dose to about 10 mg/Kg/dose. Most preferably, the therapeutically effective amount may be from about 0.01 mg/Kg/dose to about 5 mg/Kg/dose. Therefore, the therapeutically effective amount of the active ingredient contained per dosage unit (e.g., tablet, capsule, powder, injection, suppository, teaspoonful and the like) as described herein may be from about 1 mg/day to about 7000 mg/day for a subject, for example, having an average weight of 70 Kg.

The dosages, however, may be varied depending upon the requirement of the subjects (including factors associated with the particular subject being treated, including subject age, weight and diet, strength of the preparation, the advancement of the disease condition and the mode and time of administration) and the use of a particular compound of Formula (I) or Formula (II) or pharmaceutical composition thereof.

Optimal dosages to be administered may be readily determined by those skilled in the art and will result in the need to adjust the dose to an appropriate therapeutic level. The use of either daily administration or post-periodic dosing may be employed. Preferably, a compound of Formula (I) or Formula (II) or pharmaceutical composition thereof for preventing or treating neurodegenerative disorders is administered orally or parenterally.

In accordance with the present invention, a compound of Formula (I) or Formula (II) or pharmaceutical composition thereof described herein may be administered separately, at different times during the course of therapy or concurrently in divided combination or single combination forms. Advantageously, a compound selected from the group consisting of Formula (I) and Formula (II) or pharmaceutical compositions thereof may be administered in a single daily dose or the total daily dosage may be administered via continuous delivery or in divided doses of two, three or four times daily.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human, that is being sought by a researcher, veterinarian, medical doctor, or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

To prepare a pharmaceutical composition of the present invention, a compound of Formula (I) or Formula (II) as the active ingredient is intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Preferably, a pharmaceutical composition is in a unit dosage form such as a tablet, pill, capsule, caplet, gelcap, lozenge, granule, powder, sterile parenteral solution or suspension, metered aerosol or liquid spray, drop, ampoule, autoinjector device or suppository for administration by oral, intranasal, sublingual, intraocular, transdermal, parenteral, rectal, vaginal, inhalation or insufflation means. Alternatively, the composition may be presented in a form suitable for once-weekly or once-monthly administration or may be adapted to provide a preparation for intramuscular injection.

In preparing a pharmaceutical composition having a solid dosage form for oral administration, such as a tablet, pill, capsule, caplet, gelcap, lozenge, granule or powder (each including immediate release, timed release and sustained release formulations), suitable carriers and additives include but are not limited to diluents, granulating agents, lubricants, binders, glidants, disintegrating agents and the like. If desired, tablets may be sugar coated, gelatin coated, film coated or enteric coated by standard techniques.

For preparing a solid dosage form, the principal active ingredient is mixed with a pharmaceutical carrier (e.g. conventional tableting ingredients such as diluents, binders, adhesives, disintegrants, lubricants, antiadherents and glidants). Sweeteners and flavorants may be added to chewable solid dosage forms to improve the palatability of the oral dosage form. Additionally, colorants and coatings may be added or applied to the solid dosage form for ease of identification of the drug or for aesthetic purposes. These carriers are formulated with the pharmaceutical active to provide an accurate, appropriate dose of the pharmaceutical active with a therapeutic release profile.

In preparing a pharmaceutical composition having a liquid dosage form for oral, topical and parenteral administration, any of the usual pharmaceutical media or excipients may be employed. Thus, for liquid unit dosage forms, such as suspensions (i.e. colloids, emulsions and dispersions) and solutions, suitable carriers and additives include but are not limited to pharmaceutically acceptable wetting agents, dispersants, flocculation agents, thickeners, pH control agents (i.e. buffers), osmotic agents, coloring agents, flavors, fragrances, preservatives (i.e. to control microbial growth, etc.) and a liquid vehicle may be employed. Not all of the components listed above will be required for each liquid dosage form. The liquid forms in which the novel compositions of the present invention may be incorporated for administration orally or by injection include, but are not limited to aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles.

### Biological Experimental Examples

The activities of a compound of Formula (I) and Formula (II) for use in preventing or treating neurodegenerative disorders were evaluated in the following experimental example which is intended to be a way of illustrating but not limiting the invention.

### Example 1

### PC12 Cell Serum Withdrawal Model

Serum withdrawal is a cytotoxic environmental challenge that results in cell death in cultured cell lines as well as in primary cells of various tissue origins, including nerve cells. In particular, pheochromocytoma (PC) 12 cells have been widely employed as an in vitro neuronal cell model for a wide variety of neurodegenerative and cell death related disorders (Muriel, et al, Mitochondrial free calcium levels (Rhod-2 fluorescence) and ultrastructural alterations in neuronally differentiated PC12 cells during ceramide-dependent cell death, J. *Comp. Neurol.,* **2000**, 426(2), 297-315; Dermitzaki, et al, Opioids transiently prevent activation of apoptotic mechanisms following short periods of serum withdrawal, *J. Neurochem.,* **2000,** 74(3), 960-969; Carlile, et al, Reduced apoptosis after nerve growth factor and serum withdrawal: conversion of tetrameric glyceraldehyde-3-phosphate dehydrogenase to a dimer, *Mol. Pharmacol.,* **2000,** 57(1), 2-12).

PC12 cells were cultured in sterile media (RPMI 1640) supplemented with 10% heat-inactivated horse serum and 5% fetal bovine serum (FBS). The culture medium also contained 1 X Penicillin-Streptomycin-Neomycin antibiotic (50 µg, 50 µg, 100 µg, respectively). Medium was exchanged every other day and the cells were passed in log phase near confluence.

The control cells were cultured in regular media without any treatment. An enantiomer of Formula (Ib) or Formula (IIb) (10 µM) was mixed well in the medium and then applied to the cells. For the 2 day assay, an enantiomer of Formula (Ib) or Formula (IIb) (10 µM) was only applied to the cells once at the time of serum withdrawal. For the 7 day assay, an enantiomer of Formula (Ib) or Formula (IIb) (10 µM) was applied to the cells at the time of serum withdrawal and every 48 hr thereafter when cells were changed with fresh new serum-free medium. In the serum withdrawal group, the cells were cultured in serum-free medium with no additional enantiomer of Formula (Ib) or Formula (IIb). Cell survival was determined by the 3-(4, 5-dimethylthiazol-2-yl)-5-(3-carboxy-methoxyphenyl)-2-(4-sulfophenyl) -2H-tetrazolium inner salt (MTS) assay at 2 or 7 days after serum withdrawal.

At the end of the experiment, cells were washed with fresh medium and incubated with MTS solution in a humidified 37°C with 5% CO2 incubator for 1.5 hr. After the incubation period, the cells were immediately analyzed using a Softmax program (Molecular Devices). MTS assay is a calorimetric method for determining the number of viable cells in a given experimental setting. The assay is based on the cellular conversion of the tetrazolium salt, MTS, into a formazan that is soluble in tissue culture medium and measured directly at 490 nm in 96-well assay plates. The absorbance is directly proportional to the number of living cells in culture. The arbitrary absorbance reading in control cells is expressed as 100% survival rate.

Table 1 lists data demonstrating the effect on cell survival rate of the orally administered enantiomer of Formula (Ib) and Formula (IIb) in the PC12 cell serum withdrawal model (¹p value = 0.01; ²p value = <0.01).

**Table 1**

| **% Cell Survival Rate** | | |
|---|---|---|
| | **2 Day Survival Rate (%)** | **7 Day Survival Rate (%)** |
| **Control** | 100 | 100 |
| **Serum-free** | 49.6 ± 2.6 | 23.8 ± 2.6 |
| **Formula (Ib)** | 69.4 ± 1.7¹ | 79.9 ± 4.0² |
| **Formula (IIb)** | 66.4 ± 5.4¹ | 85.2 ± 0.6² |

### Example 2

### The Transient Cerebral Ischemia Rat Model

An enantiomer of Formula (Ib) was investigated in the transient cerebral ischemia middle cerebral artery occlusion (MCAO) rat model (as described in Nagasawa H. and Kogure K., *Stroke,* **1989**, 20, 1037; and, Zea Longa E., Weinstein P.R., Carlson S. and Cummins R., *Stroke,* **1989**, 20, 84) using male Wistar rats at 10 and 100 mg/kg (i.v.). MK 801 (Dizocilpine maleate; CAS Registry number 77086-22-7, a commercially available compound) was used as a positive control (3 mg/kg, i.p.).

Rats (n = 12) were randomly allocated to one of four experimental groups and were anesthetized. Blood flow from the internal carotid artery, anterior cerebral artery and posterior cerebral artery into the middle cerebral artery was blocked by this procedure. One hour after blockage, animals were treated over a 1 hour period with vehicle (administered i.v. over the one hour period), control (administered as a single i.p. dose at the start of the one hour period) and two doses of an enantiomer of Formula (Ib) (administered i.v. over the one hour period). Two hours after blockage, reperfusion was performed.

The animals were sacrificed and 20mm-thick coronal sections of each brain were prepared. One in every forty sections (i.e. every 800 nM) from the front to the occipital cortex was used to quantify the extent of the cerebral lesion. Slides were prepared using sections stained (according to the Nissl procedure) with cresyl violet and were examined under a light microscope.

Regional ischemic surface areas in the coronal sections of individual rats were determined according to the presence of cells with morphological changes. The areas of neuronal injury or infarction were measured and then added. The cortex and striatum volume were calculated for each animal (total ischemic surface area x 0.8 mm (thickness)).

### MCAO Model Analysis

The mean volumes (± S.E.M.) for each animal randomly assigned to the four experimental groups were compared using one-way ANOVA (one way ANOVA is a statistical method which compares 3 or more unmatched groups) followed by Dunnett's t-test (both methods incorporated in Statview 512+ software, BarinPower, Calabasas, CA, USA).

As shown in Table 2, results were considered statistically significant when the p value was < 0.05 compared to vehicle group (¹p<0.01; ²p<0.05).

**Table 2**

| | **Mean Infarct Volume (mm³) ± S.E.M.** | | | |
|---|---|---|---|---|
| **Treatment** | **N** | **Cortex** | **Striatum** | **Total Volume** |
| Vehicle, 10 mUkg | 12 | 275.5 ± 27.1 | 79.4 ± 3.6 | 354.9 ± 29.9 |
| MK 801, 3 mg/kg | 12 | 95.8 ± 24.5¹ | 56.1 ± 5.3² | 151.9 ± 28.7¹ |
| Formula (Ib), 10 mg/kg | 12 | 201.0 ± 23.9 | 75.9 ± 2.6 | 276.9 ± 25.4 |
| Formula (Ib), 100 mg/kg | 12 | 98.8 ± 29.5¹ | 63.0 ± 5.9² | 161.9 ± 34.3¹ |

## Claims

1. Use of a compound selected from the group consisting of Formula (I) and Formula (II): 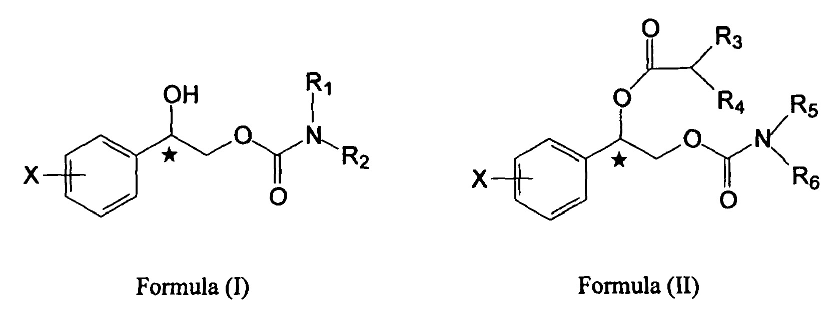 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano);
for the manufacture of a medicament for preventing or treating neurodegenerative disorders.

2. The use of claim 1 wherein X is chlorine.

3. The use of claim 1 wherein X is substituted at the ortho position of the phenyl ring.

4. The use of claim 1 wherein R₁, R₂, R₃, R₄, R₅ and R₆ are selected from hydrogen.

5. Use of an enantiomer selected from the group consisting of Formula (I) and Formula (II) or enantiomeric mixture wherein one enantiomer selected from the group consisting of Formula (I) and Formula (II) predominates: 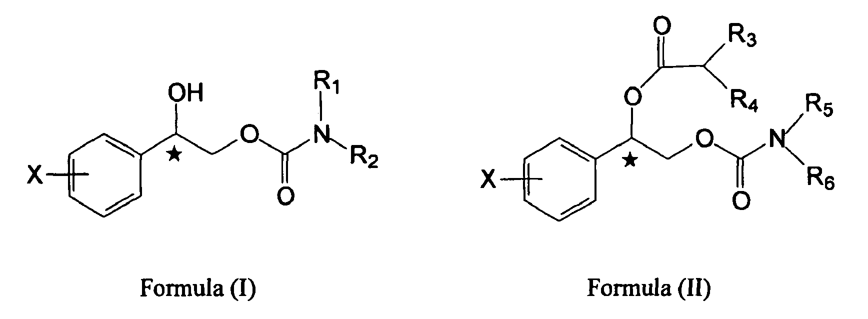 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano);
for the manufacture of a medicament for preventing or treating neurodegenerative disorders.

6. The use of claim 5 wherein the enantiomer selected from the group consisting of Formula (I) and Formula (II) is an enantiomer selected from the group consisting of Formula (Ib) and Formula (IIb):

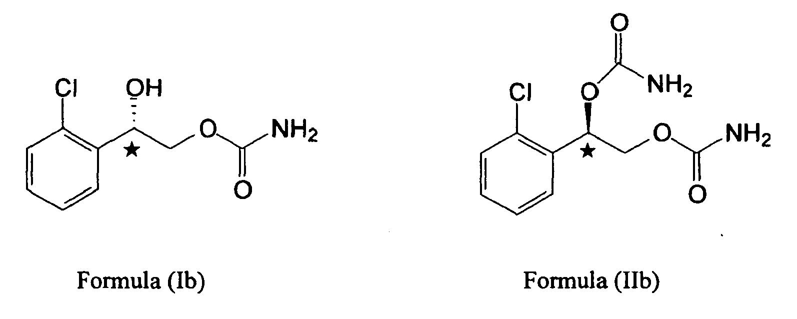

7. The use as in claims 1 or 5 wherein neurodegenerative disorders are selected from the group consisting of acute neurodegenerative disorders, chronic neurodegenerative disorders, other acute or chronic neurodegenerative disorders associated with memory loss and other acute or chronic neurodegenerative disorders associated with neuronal injury.

8. The use of claim 7 wherein acute neurodegenerative disorders are selected from neurodegenerative disorders associated with an abrupt insult selected from acute injury, hypoxia-ischemia or the combination thereof resulting in neuronal cell death or compromise.

9. The use of claim 8 wherein acute injury is selected from brain trauma, focal brain trauma, diffuse brain damage, spinal cord injury, intracranial lesions (selected from contusion, penetration, shear, compression or laceration lesions), intravertebral lesions (selected from contusion, penetration, shear, compression or laceration lesions) or whiplash shaken infant syndrome.

10. The use of claim 9 wherein acute injury is selected from brain trauma, focal brain trauma, diffuse brain damage or spinal cord injury.

11. The use of claim 8 wherein hypoxia-ischemia is selected from cerebrovascular insufficiency, cerebral ischemia or cerebral infarction.

12. The use of claim 11 wherein cerebral ischemia or cerebral infarction are selected from cerebral ischemias or infarctions originating from embolic occlusion, thrombotic occlusion, reperfusion following acute ischemia, perinatal hypoxic-ischemic injury, cardiac arrest or intracranial hemorrhage (wherein hemorrhage is selected from epidural, subdural, subarachnoid or intracerebral hemorrhage).

13. The use of claim 7 wherein chronic neurodegenerative disorders are selected from neurodegenerative disorders associated with progressive neuronal cell death or compromise over a period of time selected from Alzheimer's disease, Pick's disease, diffuse Lewy body disease, progressive supranuclear palsy (selected from Steel-Richardson syndrome), multisystem degeneration (selected from Shy-Drager syndrome), chronic epileptic conditions associated with neurodegeneration, motor neuron diseases (selected from amyotrophic lateral sclerosis), multiple sclerosis, degenerative ataxias, cortical basal degeneration, ALS-Parkinson's-Dementia complex of Guam, subacute sclerosing panencephalitis, Huntington's disease, Parkinson's disease, synucleinopathies (selected from multiple system atrophy), primary progressive aphasia, striatonigral degeneration, Machado-Joseph disease/spinocerebellar ataxia type 3 and olivopontocerebellar degenerations, bulbar and pseudobulbar palsy, spinal and spinobulbar muscular atrophy (selected from Kennedy's disease), primary lateral sclerosis, familial spastic paraplegia, Werdnig-Hoffinann disease, Kugelberg-Welander disease, Tay-Sach's disease, Sandhoff disease, familial spastic disease, Wohlfart-Kugelberg-Welander disease, spastic paraparesis, progressive multifocal leukoencephalopathy, familial dysautonomia (selected from Riley-Day syndrome) or prion diseases (selected from Creutzfeldt-Jakob disease, Gerstmann-Sträussler-Scheinker disease, Kuru disease or fatal familial insomnia).

14. The use of claim 13 wherein chronic neurodegenerative disorders are selected from Alzheimer's disease, chronic epileptic conditions associated with neurodegeneration, multiple sclerosis or Parkinson's disease.

15. The use of claim 7 wherein other acute or chronic neurodegenerative disorders associated with memory loss are selected from neurodegenerative disorders associated with age-related dementia, vascular dementia, diffuse white matter disease (selected from Binswanger's disease), dementia of endocrine or metabolic origin, dementia of head trauma or diffuse brain damage, dementia pugilistica or frontal lobe dementia.

16. The use of claim 7 wherein other acute or chronic neurodegenerative disorders associated with neuronal injury are selected from neurodegenerative disorders associated with chemical, toxic, infectious and radiation injury of the nervous system, injury during fetal development, prematurity at time of birth, anoxic-ischemia, injury from hepatic, glycemic, uremic, electrolyte and endocrine origin, injury of psychiatric origin, injury from peripheral diseases and plexopathy (selected from plexus palsies) or injury from neuropathy.

17. The use of claim 16 wherein other acute or chronic neurodegenerative disorders associated with neuronal injury are selected from neurodegenerative disorders associated with injury of psychiatric origin or injury from neuropathy.

18. The use of claim 17 wherein injury of psychiatric origin is selected from psychopathology, depression or anxiety; and, wherein injury from neuropathy is selected from multifocal, sensory, motor, sensory-motor, autonomic, sensory-autonomic or demyelinating neuropathies (selected from Guillain-Barre syndrome or chronic inflammatory demyelinating polyradiculoneuropathy) or those neuropathies originating from infections, inflammation, immune disorders, drug abuse, pharmacological treatments, toxins, trauma (selected from compression, crush, laceration or segmentation traumas), metabolic disorders (selected from endocrine or paraneoplastic), Charcot-Marie-Tooth disease (selected from type 1a, 1b, 2, 4a or 1-X linked), Friedreich's ataxia, metachromatic leukodystrophy, Refsum's disease, adrenomyeloneuropathy, Ataxia-telangiectasia, Dejerine-Sottas (selected from types A or B), Lambert-Eaton syndrome or disorders of the cranial nerves).

## Patentansprüche

1. Verwendung einer Verbindung, die ausgewählt ist aus der Gruppe, bestehend aus Formel (I) und Formel (II): 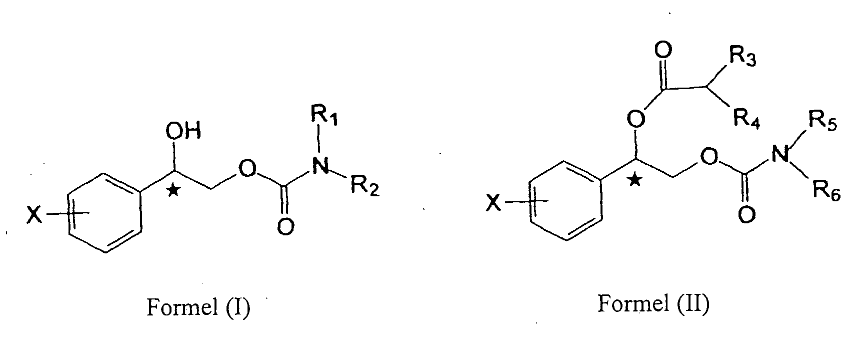 worin
Phenyl bei X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl; wobei C₁-C₄-Alkyl fakultativ mit Phenyl substituiert ist (wobei Phenyl fakultativ mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano);
zur Herstellung eines Arzneimittels zur Vorbeugung oder Behandlung von neurodegenerativen Störungen.

2. Verwendung nach Anspruch 1, **dadurch gekennzeichnet, daß** X Chlor ist.

3. Verwendung nach Anspruch 1, **dadurch gekennzeichnet, daß** X an der ortho-Position des Phenylrings substituiert ist.

4. Verwendung nach Anspruch 1, **dadurch gekennzeichnet, daß** R₁, R₂, R₃, R₄, R₅ und R₆ aus Wasserstoff ausgewählt sind.

5. Verwendung eines Enantiomers, das ausgewählt ist aus der Gruppe, bestehend aus Formel (I) und Formel (II), oder einer Enantiomerenmischung, in der ein Enantiomer, das ausgewählt ist aus der Gruppe, bestehend aus Formel (I) und Formel (II), überwiegt: 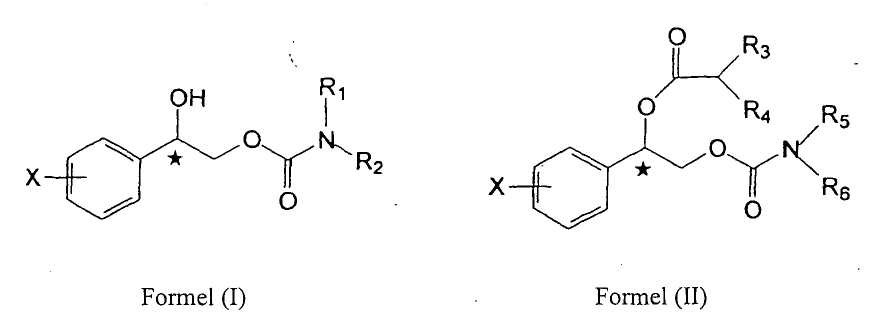 worin
Phenyl bei X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl, wobei C₁-C₄-Alkyl fakultativ mit Phenyl substituiert ist (wobei Phenyl fakultativ mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano);
zur Herstellung eines Arzneimittels zur Vorbeugung oder Behandlung von neurodegenerativen Störungen.

6. Verwendung nach Anspruch 5, **dadurch gekennzeichnet, daß** das Enantiomer, das ausgewählt ist aus der Gruppe, bestehend aus Formel (I) und Formel (II), ein Enantiomer ist, das ausgewählt ist aus der Gruppe, bestehend aus Formel (Ib) und Formel (IIb):

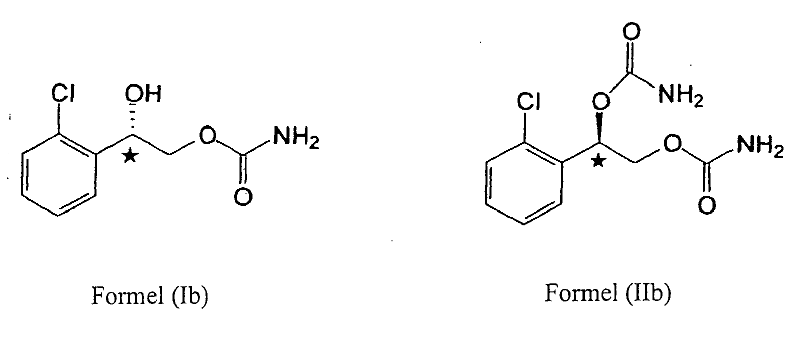

7. Verwendung wie in den Ansprüchen 1 oder 5, **dadurch gekennzeichnet, daß** die neurodegenerativen Störungen ausgewählt sind aus der Gruppe, bestehend aus akuten neurodegenerativen Störungen, chronischen neurodegenerativen Störungen, anderen akuten oder chronischen neurodegenerativen Störungen, die verbunden sind mit Gedächtnisverlust, oder anderen akuten oder chronischen neurodegenerativen Störungen, die verbunden sind mit neuronaler Verletzung.

8. Verwendung nach Anspruch 7, **dadurch gekennzeichnet, daß** die akuten neurodegenerativen Störungen ausgewählt sind aus neurodegenerativen Störungen, die mit einem abruptem Insult verbunden sind, ausgewählt aus akuter Verletzung, Hypoxie-Ischämie oder der Kombination davon, was zu neuronalem Zelltod oder -beeinträchtigung führt.

9. Verwendung nach Anspruch 8, **dadurch gekennzeichnet, daß** die akute Verletzung ausgewählt ist aus Hirntrauma, fokalem Hirntrauma, diffuser Hirnschädigung, Rückenmarkverletzung, intrakranialen Läsionen (ausgewählt aus Kontusions-, Penetrations-, Scher-, Kompressions- oder Lazerationsläsionen) oder Peitschenschlagsyndrom bei Kleinkindern.

10. Verwendung nach Anspruch 9, **dadurch gekennzeichnet, daß** die akute Verletzung ausgewählt ist aus Hirntrauma, fokalem Hirntrauma, diffuser Himschädigung oder Rückenmarkverletzung.

11. Verwendung nach Anspruch 8, **dadurch gekennzeichnet, daß** die Hypoxie-Ischämie ausgewählt ist aus zerebrovaskulärer Insuffizienz, zerebraler Ischämie oder zerebralem Infarkt.

12. Verwendung nach Anspruch 11, **dadurch gekennzeichnet, daß** die zerebrale Ischämie oder der zerebrale Infarkt ausgewählt sind aus zerebralen Ischämien oder Infarkten, die von Embolieverschluß, Thromboseverschluß, Reperfusion im Anschluß an akute Ischämie, perinataler hypoxisch-ischämischer Verletzung, Herzstillstand oder interkranialer Hämorrhagie (wobei die Hämorrhagie ausgewählt aus epiduraler, subduraler, subarachnoider oder intrazerebraler Hämorrhagie) herrühren.

13. Verwendung nach Anspruch 7, **dadurch gekennzeichnet, daß** die chronischen neurogenerativen Störungen ausgewählt sind aus neurodegenerativen Störungen, die mit progressiven neuronalen Zelltod oder -beeinträchtigung über einen bestimmten Zeitraum verbunden sind, ausgewählt aus Alzheimer-Krankheit, Pick-Krankheit, diffuser Lewy-Körperchen-Krankheit, progressiver supranukleärer Paralyse (ausgewählt aus Steel-Richardson-Syndrom), Multisystemdegeneration (ausgewählt aus Shy-Drager-Syndrom), chronischen epileptischen Zuständen, die mit Neurodegeneration verbunden sind, Motoneuronen-Krankheiten (ausgewählt aus amyotropher Lateralsklerose), multipler Sklerose, degenerativen Ataxien, kortikaler basaler Degeneration, ALS-Parkinson-Demenz-Komplex von Guam, subakuter sklerosierender Panencephalitis, Huntington-Krankheit, Parkinson-Krankheit, Synukleinopathien (ausgewählt aus multipler Systematrophie), primärer progressiver Aphasie, striatonigraler Degeneration, Machado-Joseph-Krankheit/spinozerebellarer Ataxie Typ 3 und olivopontozerebellarer Degenerationen, Bulbär- und Pesudobulbärparalyse, spinaler und spinobulbärer Muskelatrophie (ausgewählt aus Kennedy-Krankheit), primärer Lateralsklerose, familiärer spastischer Paraplegie, Werdnig-Hoffmann-Krankheit, Kugelberg-Welander-Krankheit, Tay-Sach-Krankheit, Sandhoff-Krankheit, familiärer spastischer Krankheit, Wohlfart-Kugelberg-Welander-Krankheit, spastischer Paraparesis, progressiver multifokaler Leukoencephalopathie, familiärer Dysautonomie (ausgewählt aus Riley-Day-Syndrom) oder Prionen-Krankheiten (ausgewählt aus Creutzfeldt-Jakob-Krankheit, Gerstmann-Sträussler-Scheinker-Krankheit, Kuru-Krankheit oder tödlicher familiärer Insomnie).

14. Verwendung nach Anspruch 13, **dadurch gekennzeichnet, daß** die chronischen neurodegenerativen Störungen ausgewählt sind aus Alzheimer-Krankheit, chronischen epileptischen Zuständen, die mit Neurodegeneration verbunden sind, multipler Sklerose oder Parkinson-Krankheit.

15. Verwendung nach Anspruch 7, **dadurch gekennzeichnet, daß** die anderen akuten oder chronischen neurodegenerativen Störungen, die mit Gedächtnisverlust verbunden sind, ausgewählt sind aus neurodegenerativen Störungen, die mit altersbedingter Demenz, vaskulärer Demenz, diffuser Krankheit der weißen Hirnsubstanz (ausgewählt aus Binswanger-Krankheit), Demenz endokrinen oder metabolischen Ursprungs, Demenz durch Kopftrauma oder diffuse Hirnschädigung, Dementia pugilistica oder Vorderlappendemenz verbunden sind.

16. Verwendung nach Anspruch 7, **dadurch gekennzeichnet, daß** die anderen akuten oder chronischen neurodegenerativen Störungen, die mit neuronaler Verletzung verbunden sind, ausgewählt sind aus neurodegenerativen Störungen, die mit chemischer, toxischer, infektiöser oder Strahlungsverletzung des Nervensystems, Verletzung während der Fötusentwicklung, Frühreife zum Zeitpunkt der Geburt, Anoxie-Ischämie, Verletzung hepatischen, glykämischen, urämischen, elektrolytischen und endokrinen Ursprungs, Verletzung psychiatrischen Ursprungs, Verletzung durch periphere Krankheiten und Plexopathie (ausgewählt aus Plexusparalyse) oder Verletzung durch Neuropathie verbunden sind.

17. Verwendung nach Anspruch 16, **dadurch gekennzeichnet, daß** die anderen akuten oder chronischen neurodegenerativen Störungen, die mit neuronaler Verletzung verbunden sind, ausgewählt sind aus neurodegenerativen Störungen, die mit Verletzung psychiatrischen Ursprungs oder Verletzung durch Neuropathie verbunden sind.

18. Verwendung nach Anspruch 17, **dadurch gekennzeichnet, daß** die Verletzung psychiatrischen Ursprungs ausgewählt ist aus Psychopathologie, Depression oder Angstzustand; und daß die Verletzung durch Neuropathie ausgewählt ist aus multifokalen, sensorischen, motorischen, sensorisch-motorischen, autonomen, sensorisch-autonomen oder demyelinierenden Neuropathien (ausgewählt aus Guillain-Barre-Syndrom oder chronischer enzündlicher demyelinierender Polyradiculoneuropathie) oder denjenigen Neuropathien, die von Infektionen, Entzündungen, Immunstörungen, Medikamentenmißbrauch, pharmakologischen Behandlungen, Toxinen, Trauma (ausgewählt aus Kompressions-, Crush-, Lazerations- oder Segmentationstraumata), metabolischen Störungen (ausgewählt aus endokrinen oder paraneoplastischen Störungen), Charcot-Marie-Tooth-Krankheit (ausgewählt aus Typ 1a, 1b, 2, 4a oder 1-X-verknüpft), Friedreich-Ataxie, metachromatischer Leukodystrophie, Refsum-Krankheit, Adrenomyeloneuropathie, Ataxie-Telangiectasie, Déjerine-Sottas (ausgewählt aus Typ A oder B), Lambert-Eaton-Syndrom oder Störungen der Kranialnerven herrührt.

## Revendications

1. Utilisation d'un composé sélectionné dans le groupe consistant en Formule (I) et Formule (II): 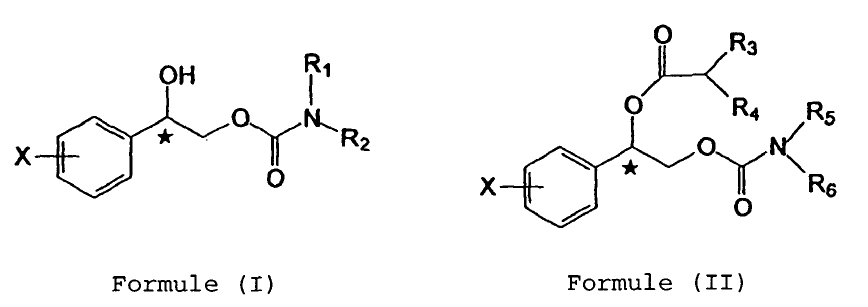 où
phényle est substitué en X par un à cinq atomes d'halogène sélectionnés dans le groupe consistant en fluor, chlore, brome et iode; et
R₁, R₂, R₃, R₄, R₅ et R₆ sont indépendamment sélectionnés dans le groupe consistant en hydrogène et alkyle C₁-C₄; où alkyle C₁-C₄ est facultativement substitué par phényle (où phényle est facultativement substitué par des substituants indépendamment sélectionnés dans le groupe consistant en halogène, alkyle C₁-C₄, alcoxy C₁-C₄, amino, nitro et cyano); pour la fabrication d'un médicament pour prévenir ou traiter des maladies neurodégénératives.

2. Utilisation de la revendication 1 où X est chlore.

3. Utilisation de la revendication 1 où X est substitué à la position ortho du cycle phényle.

4. Utilisation de la revendication 1 où R₁,R₂,R₃,R₄,R₅ et R₆ sont sélectionnés parmi de l'hydrogène.

5. Utilisation d'un énantiomère sélectionné dans le groupe consistant en Formule (I) et Formule (II) ou un mélange d'énantiomères où un énantiomère sélectionné dans le groupe consistant en Formule (I) et Formule (II) prédomine: 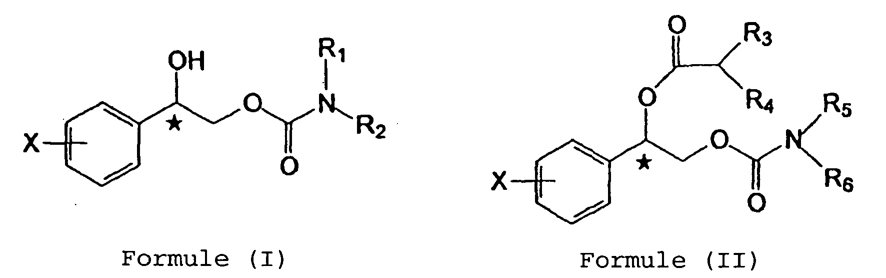 où
phényle est substitué en X par un à cinq atomes d'halogène sélectionnés dans le groupe consistant en fluor, chlore, brome et iode; et
R₁,R₂,R₃,R₄,R₅ et R₆ sont indépendamment sélectionnés dans le groupe consistant en hydrogène et alkyle C₁-C₄; où alkyle C₁-C₄ est facultativement substitué par phényle (où phényle est facultativement substitué par des substituants indépendamment sélectionnés dans le groupe consistant en halogène, alkyle C₁-C₄, alcoxy C₁-C₄, amino, nitro et cyano); pour la fabrication d'un médicament pour prévenir ou traiter des maladies neurodégénératives.

6. Utilisation de la revendication 5 où l'énantiomère sélectionné dans le groupe consistant en Formule (I) et Formule (II) est un énantiomère sélectionné dans le groupe consistant en Formule (Ib) et Formule (IIb):

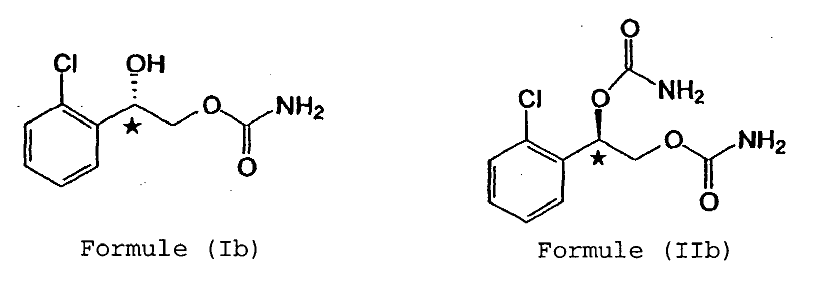

7. Utilisation selon les revendications 1 ou 5 où les troubles neurodégénératifs sont sélectionnés dans le groupe consistant en troubles neurodégénératifs aigus, troubles neurodégénératifs chroniques, autres troubles dégénératifs aigus ou chroniques associés à la perte de mémoire et autres troubles neurodégénératifs aigus ou chroniques associés à une lésion des neurones.

8. Utilisation de la revendication 7 où les troubles neurodégénératifs aigus sont sélectionnés parmi des troubles neurodégénératifs associés à une insulte abrupte sélectionnée parmi une lésion aiguë, une hypoxie-ischémie ou une combinaison de celles-ci avec pour résultat une cellule neuronale morte ou compromise.

9. Utilisation de la revendication 8 où la lésion aiguë est sélectionnée parmi un traumatisme du cerveau, un traumatisme focal du cerveau, un traumatisme diffus du cerveau, une lésion dé la moelle épinière, des lésions intracraniennes (sélectionnées parmi les ions par contusion, pénétration, cisaillement, compression ou lacération), des lésions intravertébrales (sélectionnées parmi lésions par contusion, pénétration, cisaillement, compression et lacération) ou syndrome du nourrisson secoué.

10. Utilisation de la revendication 9 où la lésion aiguë est sélectionnée parmi un traumatisme du cerveau, un traumatisme focal du cerveau, une dégradation diffuse du cerveau ou une lésion de la moelle épinière.

11. Utilisation de la revendication 8 où l'hypoxie-ischémie est sélectionnée parmi une insuffisance cérébrovasculaire, une ischémie cérébrale ou un infarctus cérébral.

12. Utilisation de la revendication 11 où l'ischémie cérébrale ou l'infarctus cérébral sont sélectionnés parmi des ischémies cérébrales ou des infarctus provenant d'une occlusion embolique, d'une occlusion thrombotique, d'une reperfusion à la suite d'une ischémie aiguë, d'une lésion hypoxique-ischémique périnatale, d'un arrêt cardiaque ou d'une hémorragie intracranienne (où l'hémorragie est sélectionnée parmi hémorragie épidurale, subdurale, subarachnoïde ou intracérébrale.

13. Utilisation de la revendication 7 où les troubles chroniques neurodégénératifs sont sélectionnés parmi des troubles neurodégénératifs associés à une mort progressive des cellules des neurones ou leur état compromis sur une période de temps, sélectionnée parmi la maladie de Alzheimer, la maladie de Pick, une maladie des corps de Lewy diffus, la paralysie supranucléaire progressive (sélectionnée parmi le syndrome de Steel-Richardson), une dégénérescence multisystème (sélectionnée parmi le syndrome de Shy-Drager), des conditions épileptiques chroniques associées à une neurodégénérescence, des maladies motrices des neurones (sélectionnées parmi la sclérose latérale amyotrophe), la sclérose en plaque, les ataxies dégénératives, la dégénérescence basale corticale, un complexe de Guam de ALS-Parkinson-Démence, une panencéphalite sclérosante sub-aiguë, la maladie de Huntington, la maladie de Parkinson, des synucléinopathies (sélectionnées parmi une atrophie multiple du système), une aphasie progressive primaire, une dégénérescence striatonigrale, une attaque du type 3 spinocérébelleuse/maladie de Machado-Joseph et des dégénérescences olivopontocérébelleuses, une paralysie bulbaire et pseudobulbaire, une atrophie musculaire spinale et spinobulbaire (sélectionnée parmi la maladie de Kennedy), une sclérose latérale primaire, une paraplégie spasmodique familiale, la maladie de Werdnig-Hoffmann, la maladie de Kugelberg-Welander, la maladie de Tay-Sach, la maladie de Sandhoff, la maladie spasmodique familiale, la maladie de Wohlfart-Kugelberg-Welander, la paraplésie spasmodique, la leucoencéphalopathie multifocale progressive, la dysautonomie familiale (sélectionnée parmi le syndrome de Riley-Day) ou les maladies du prion (sélectionnées parmi la maladie de Creutzfeldt-Jakob, la maladie de Gerstmann-Sträussler-Scheinker, la maladie de Kuru ou l'insomnie familiale fatale).

14. Utilisation de la revendication 13 où les troubles neurodégénératifs chroniques sont sélectionnés parmi la maladie de Alzheimer, des conditions épileptiques chroniques associées à la neurodégénérescence, la sclérose en plaques ou la maladie de Parkinson.

15. Utilisation de la revendication 7 où d'autres troubles neurogénératifs aigus ou chroniques associés à la perte de mémoire sont sélectionnés parmi des troubles neurodégénératifs associés à la démence en rapport avec l'âge, la démence vasculaire, et la maladie diffuse de la matière blanche (sélectionnée parmi la maladie de Binswanger), la démence d'origine endocrinienne ou métabolique, la démence due à un traumatisme de la tête ou une dégradation diffuse du cerveau, la démence pugilistica ou une démence du lobe frontal.

16. Utilisation de la revendication 7 où d'autres troubles neurodégénératifs aigus ou chroniques associés à une lésion des neurones sont sélectionnés parmi des troubles neurodégénératifs associés à une lésion chimique, toxique, infectieuse et par rayonnement du système nerveux, une lésion pendant le développement foetal, une prématurité au moment de la naissance, une ischémie anorexique, une lésion d'origine hépatique, glycémique, urémique, électrolytique et endocrinienne, une lésion d'origine psychiatrique, une lésion provenant de maladies périphériques et plexopathie (sélectionnée parmi les paralysies du plexus) ou une lésion provenant d'une neuropathie.

17. Utilisation de la revendication 16 où d'autres troubles neurodégénératifs aigus ou chroniques associés à une lésion des neurones sont sélectionnés parmi des troubles neurodégénératifs associés à une lésion d'origine psychiatrique ou une lésion provenant d'une neuropathie.

18. Utilisation de la revendication 17 où la lésion d'origine psychiatrique est sélectionnée parmi une psychopathologie, une dépression ou l'anxiété; et où la lésion provenant d'une neuropathie est sélectionnée parmi des neuropathies multifocales sensorielles, motrices, sensorielles-motrices, autonomes, sensorielles-autonomes ou démyélinantes (sélectionnées parmi le syndrome de Guillain-Barre ou la polyradiculoneuropathie démyélinante inflammatoire chronique) ou bien les neuropathies provenant d'infections, d'une inflammation, de troubles immuns, d'un abus de drogues, de traitements pharmacologiques, de toxines, d'un traumatisme (sélectionné parmi traumatisme par compression, écrasement, lacération ou segmentation), de troubles métaboliques (sélectionnés parmi endocrinien ou paranéoplasique), la maladie de Charcot-Marie-Tooth (sélectionnée parmi type Ia, Ib, 2,4a ou I-X en enchaînement), l'ataxie de Friedreich, la leucodystrophie métachromatique, la maladie de Refsum, l'adrénomyéloneuropathie, l'Ataxie-télangiectasie, Déjerine-Sottas (sélectionnée parmi types A ou B), le syndrome de Lambert-Eaton ou des troubles des nerfs craniens).
